# EUROPÄISCHE PATENTSCHRIFT

(11) **EP 1 652 835 B1**
(45) Veröffentlichungstag und Bekanntmachung des Hinweises auf die Patenterteilung: **17.03.2010**
(21) Anmeldenummer: 05022494.8
(22) Anmeldetag: 14.10.2005
(51) Int. Cl.: C07C 209/78, C07C 263/10, C08G 18/76

(54) **Verfahren zur Herstellung von Di-und Polyaminen der Diphenylmethanreihe**
Process for the preparation of di- and polyamines of the diphenylmethane series
Procédé de préparation de di- et polyamines de la série du diphénylméthane

(30) Priorität: 28.10.2004 DE 102004052370
(43) Veröffentlichungstag der Anmeldung: 03.05.2006
(73) Patentinhaber: Bayer MaterialScience AG, 51368 Leverkusen (DE)
(72) Erfinder: Müller, Heinz-Herbert, Dr., 47809 Krefeld (DE); Wershofen, Stefan, Dr., 41065 Mönchengladbach (DE); Grabowski, Stefan, Dr., B-9120 Melsele (BE)

(56) Entgegenhaltungen:
- EP-A- 1 288 190
- TWITCHETT H J: "CHEMISTRY OF THE PRODUCTION OF ORGANIC ISOCYANATES" CHEMICAL SOCIETY REVIEWS, CHEMICAL SOCIETY, LONDON, GB, Bd. 3, Nr. 2, 1974, Seiten 209-230, XP008045030 ISSN: 0306-0012
- MARK H F ET AL: "METHYLENEDIANILINE" KIRK - OTHMER ENCYCLOPEDIA OF CHEMICAL TECHNOLOGY. ALKOXIDES, METAL, TO ANTIBIOTICS (PEPTIDES), NEW YORK, WILEY & SONS, US, Bd. VOL. 2, 1978, Seiten 338-348, XP008059341

## Beschreibung

Die Erfindung betrifft ein Verfahren zur Herstellung von Di- und Polyaminen der Diphenylmethanreihe.

Unter Di- und Polyaminen der Diphenylmethanreihe werden Verbindungen und Verbindungsgemische folgenden Typs verstanden: 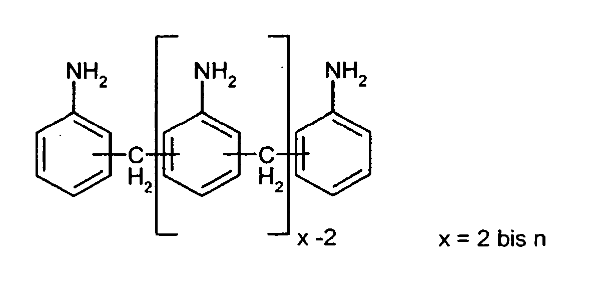 wobei n für eine natürliche Zahl > 2 steht.

Die kontinuierliche, diskontinuierliche oder halbkontinuierliche Herstellung von Di- und Polyaminen der Diphenylmethanreihe, nachfolgend auch als MDA bezeichnet, ist in zahlreichen Patenten und Publikationen beschrieben (siehe z.B. H.J. Twitchett, Chem. Soc. Rev. 3(2), 209 (1974), M.V. Moore in: Kirk-Othmer Encycl. Chem. Technol., 3rd. Ed., New York, 2, 338-348 (1978)). Üblicherweise erfolgt die Herstellung durch Umsetzung von Anilin und Formaldehyd in Anwesenheit saurer Katalysatoren. Üblicherweise wird wässrige HCl als saurer Katalysator eingesetzt. Der saure Katalysator wird gemäß dem Stand der Technik zum Ende des Prozesses und vor den abschließenden Aufarbeitungsschritten (wie beispielsweise der destillativen Entfernung von überschüssigem Anilin) durch Zusatz einer Base neutralisiert und somit verbraucht.

Die Di- und Polyisocyanate der Diphenylmethanreihe, nachfolgend als MDI bezeichnet, werden durch Phosgenierung der entsprechenden Di- und Polyamine hergestellt. Die so hergestellten Di- und Polyisocyanate der Diphenylmethanreihe enthalten dabei die verschiedenen Isocyanat-Isomeren und deren höhere Homologe in der gleichen Zusammensetzung wie die Polyamine, aus denen sie hergestellt wurden.

Im Zuge der MDA-Herstellung wird das saure Reaktionsgemisch anschließend üblicherweise mit einer Base neutralisiert. Im Anschluss an die Neutralisation wird in einem Trennbehälter üblicherweise die organische von der wässrigen Phase getrennt. Die nach Abtrennung der wässrigen Phase verbleibende produktenthaltende organische Phase wird dann üblicherweise weiteren Aufarbeitungsschritten wie einer Wäsche mit Wasser unterzogen und anschließend von überschüssigem Anilin und anderen im Gemisch vorhandenen Stoffen (z.B. weiteren Lösungsmitteln) beispielsweise durch Destillation, Extraktion oder Kristallisation befreit.

Die betriebliche Erfahrung zeigt jedoch, dass die Abtrennung der wässrigen Phase von der produktenthaltenden organischen Phase nach der Neutralisation und/oder der nachfolgenden Wäsche durch die Ausbildung einer dritten Phase (Mulm bzw. Mulmschicht) sehr stark beeinträchtigt werden kann. Diese dritte Phase ist eine stabile, ggf. voluminöse Zwischenphase, die zwischen der wässrigen und der organischen Phase auftritt und die Phasentrennung erschwert und im Extremfall sogar vollständig verhindert. Im für den betrieblichen Ablauf ungünstigsten Fall müssen der oder die betroffenen Phasentrennbehälter vollständig entleert und gereinigt werden. Der Inhalt des bzw. der Phasentrennbehälter ist dann aufwändig aufzuarbeiten oder zu entsorgen, was mit erheblichen Kosten verbunden ist. Dies kann unter Umständen auch dazu führen, dass die kontinuierliche Produktion unterbrochen werden muss.

Aufgabe der vorliegenden Erfindung war es daher, ein einfaches und wirtschaftliches Verfahren zur Herstellung von Di- und Polyaminen der Diphenylmethanreihe bereit zu stellen, bei dem die Abtrennung der wässrigen Phase von der produktenthaltenden organischen Phase nach der Neutralisation und/oder der nachfolgenden Wäsche einfach und störungsfrei durchgeführt werden kann.

Die Erfindung betrifft ein Verfahren zur Herstellung von Di- und Polyaminen der Diphenylmethanreihe, bei dem man Anilin und Formaldehyd in Gegenwart von Salzsäure umsetzt, dadurch gekennzeichnet, dass die eingesetzte Salzsäure weniger als 0,001 Gew.% an zwei- und /oder höherwertigen Metallionen enthält.

Dabei bezieht sich die Grenze von < 0,001 Gew.% auf die Summe der Konzentrationen der zwei- und / oder höherwertigen Metallionen. Bevorzugt enthält die eingesetzte Salzsäure < 0,0005 Gew.-% (0 bis < 0,0005 Gew.-%), besonders bevorzugt < 0,0003 Gew.-% (0 bis < 0,0003 Gew.-%) an zwei- und /oder höherwertigen Metallionen.

Die für die MDA-Herstellung benötigte Salzsäure kann aus unterschiedlichen Quellen bzw. Verfahren stammen. Beispielsweise kann der bei der Umsetzung von Aminen mit Phosgen freigesetzte Chlorwasserstoff in Wasser bzw. verdünnter Salzsäure absorbiert werden. In ähnlicher Weise kann auch Chlorwasserstoff aus Chlorierungen organischer Substrate genutzt werden. Möglich ist auch der Einsatz von Salzsäure, deren Konzentration an zwei- und/oder höherwertigen Metallionen beispielsweise durch Fällung in der Form schwerlöslicher Salze auf Gehalte < 0,001 Gew.-% erniedrigt wurde. Die Salzsäure weist dabei üblicherweise eine Konzentration an HCl von 25-36 Gew.-% auf, es sind jedoch auch höhere oder niedrigere Konzentrationen möglich.

Unter zwei- und /oder höherwertigen Metallionen werden Ionen in der Oxidationsstufe ≥ +2 der Metalle verstanden, die der 2. bis 6. Hauptgruppe, der 1. bis 8. Nebengruppe, der Lanthanidenreihe oder der Actinidenreihe des Periodensystems der Elemente entstammen. Beispiele für derartige Metalle sind z.B. Erdalkalimetalle wie Magnesium und Calcium, oder auch Aluminium und Eisen. Die Konzentrationen der einzelnen Metallionen in der Salzsäure betragen dabei bevorzugt < 0,0003 Gew.-%.

Das erfindungsgemäße Verfahren kann sowohl kontinuierlich, halbkontinuierlich als auch diskontinuierlich durchgeführt werden.

Mit dem erfindungsgemäßen Verfahren können Polyamine der Diphenylmethanreihe bei Protonierungsgraden von < 15 % hergestellt werden, aber auch höhere Protonierungsgrade sind möglich. Als Protonierungsgrad wird dabei bei Salzsäure das molare Verhältnis aus der Menge an eingesetzter Salzsäure und der im Reaktionsgemisch vorhandenen molaren Menge an Aminfunktionen bezeichnet.

Geeignete Polyamingemische der Diphenylmethanreihe werden üblicherweise erhalten durch Kondensation von Anilin und Formaldehyd im molaren Verhältnis von 1,5:1 bis 20:1.

Formaldehyd wird technisch üblicherweise als wässrige Lösung eingesetzt, die in Konzentrationen von 30-50 Gew.-% vorliegt. Es ist aber auch möglich, wässrige Formaldehydlösungen anderer Konzentration oder andere Methylengruppen liefernde Verbindungen, wie z.B. Polyoxymethylenglykol, para-Formaldehyd oder Trioxan einzusetzen.

In einer Ausführungsform des erfindungsgemäßen Verfahrens wird zunächst Anilin mit Salzsäure gemischt und anschließend Formaldehyd zugegeben. Es ist aber ebenfalls möglich, Anilin, Formaldehyd und Salzsäure in einer anderen Reihenfolge oder auch gleichzeitig zu vermischen.

Das erfindungsgemäße Verfahren kann z.B. so durchgeführt werden, dass Anilin, Formaldehyd-Lösung und Salzsäure in einen Rührkessel gegeben und vermischt werden und ggf. parallel zu der stattfindenden Umsetzung ein Teil des Wassers durch destillative Abtrennung entfernt wird. Gegebenenfalls können Anilin, Formaldehyd-Lösung und Salzsäure bei einem diskontinuierlichen Verfahren auch über zeitliche Dosierprofile zugegeben werden, wobei die Wasserabtrennung während oder nach der Zugabe der Edukte, beispielsweise mittels Vakuumdestillation, stattfinden kann. Bevorzugt findet die Vermischung von Anilin, Formaldehyd-Lösung und wässrige HCl bei Temperaturen von 20 bis 60°C statt.

In einer anderen Ausführungsform werden zunächst Anilin und Formaldehyd in Abwesenheit des sauren Katalysators bei Temperaturen von 20 °C bis 100 °C, bevorzugt von 40 °C bis 100 °C, besonders bevorzugt von 60 °C bis 95 °C vermischt und zur Reaktion gebracht. Hierbei bilden sich Kondensationsprodukte aus Anilin und Formaldehyd (sogenanntes Aminal). Im Anschluss an die Aminalbildung wird das in dem Aminal enthaltene Wasser beispielsweise durch Phasentrennung oder durch andere geeignete Verfahren, beispielsweise durch Destillation, zumindest teilweise entfernt.

Die Zugabe des sauren Katalysators und die Entfernung des Wassers können beispielsweise so erfolgen, dass in einen das erzeugte Aminal enthaltenden Rührkessel wässrige HCl zugegeben wird und ggf. ein Teil des Wassers während der Umsetzung zum Kondensationsprodukt durch destillative Abtrennung entfernt wird.

In einer bevorzugten Variante dieser Ausführungsform werden zunächst Anilin und Formaldehyd in Abwesenheit des sauren Katalysators bei Temperaturen von 20 °C bis 100 °C, bevorzugt von 40 °C bis 100 °C, besonders bevorzugt von 60 °C bis 95 °C vermischt und zur Reaktion gebracht. Hierbei bilden sich Kondensationsprodukte aus Anilin und Formaldehyd (sogenanntes Aminal). Im Anschluss an die Aminalbildung wird das in dem Aminal enthaltene Wasser beispielsweise durch Phasentrennung oder durch andere geeignete Verfahren, beispielsweise durch Destillation, zumindest teilweise entfernt.

Das Aminal wird dann mit Salzsäure bevorzugt bei Temperaturen von 20 bis 60°C und bevorzugt mit spezifischen Leistungseinträgen von größer 10 kW/m³ vermischt.

Die weitere Umsetzung des gemäß einer der vorstehenden Ausführungsformen erhaltenen Reaktionsgemisches erfolgt in üblichen Reaktionsapparaten. Geeignet sind zum Beispiel gerührte Reaktoren, Rohrreaktoren oder auch Rohrreaktoren mit Einbauten wie Lochböden, welche die Verweilzeitcharakteristik im Reaktor beeinflussen. Geeignet ist auch eine Kombination aus mehreren Reaktortypen.

Bevorzugt wird die Temperatur des Reaktionsgemisches in Stufen oder kontinuierlich und ggf. unter Überdruck auf eine Temperatur von 110 °C bis 250 °C, besonders bevorzugt von 110 °C bis 180 °C, ganz besonders bevorzugt von 110 °C bis 160 °C gebracht. Die erforderliche Verweilzeit wird so gewählt, dass vollständiger Umsatz sichergestellt ist.

Die Umsetzung von Anilin und Formaldehyd in Gegenwart von Salzsäure kann auch in Anwesenheit weiterer Stoffe wie Salzen oder organischen oder anorganischen Säuren erfolgen.

Zur Aufarbeitung des sauren Reaktionsgemisches wird das Reaktionsgemisch üblicherweise mit einer Base neutralisiert. Nach dem Stand der Technik erfolgt die Neutralisation üblicherweise bei Temperaturen von beispielsweise 90 bis 100°C ohne Zusatz weiterer Substanzen. (H.J. Twitchett, Chem. Soc. Rev. 3(2), 223 (1974)). Sie kann aber auch auf einem anderen Temperaturniveau erfolgen, um z.B. den Abbau von störenden Nebenprodukten zu beschleunigen. Als Basen sind beispielsweise geeignet die Hydroxide der Alkali- und Erdalkalielemente. Vorzugsweise kommt wässrige NaOH zur Anwendung.

Die zur Neutralisation eingesetzte Base wird bevorzugt in Mengen von größer 100 %, besonders bevorzugt 105 bis 120 % der für die Neutralisation des eingesetzten sauren Katalysators stöchiometrisch benötigten Menge eingesetzt.

Im Anschluss an die Neutralisation wird in einem Trennbehälter üblicherweise die organische Phase von der wässrigen Phase getrennt. Die nach Abtrennung der wässrigen Phase verbleibende produktenthaltende organische Phase wird anschließend üblicherweise weiteren Aufarbeitungsschritten unterzogen (z.B. Wäsche mit Wasser) und anschließend von überschüssigem Anilin und anderen im Gemisch vorhandenen Stoffen (z.B. weiteren Lösungsmitteln) durch geeignete Verfahren wie z.B. Destillation, Extraktion oder Kristallisation befreit.

Durch den erfindungsgemäßen Einsatz von Salzsäure, die weniger als 0,001 Gew.-% an zwei- und /oder höherwertigen Metallionen enthält, kann die Abtrennung der wässrigen Phase von der MDA enthaltenden organischen Phase nach der Neutralisation und/oder der nachfolgenden Wäsche einfach und störungsfrei durchgeführt werden. Denn die niedrigen Gehalte an zwei- und / oder höherwertigen Metallionen in der Salzsäure bewirken, dass sich eine die Phasentrennung störende oder erschwerende dritte Phase (Mulm bzw. Mulmschicht) nicht mehr ausbildet.

Die so erhaltenen Di- und Polyamine können nach den bekannten Methoden mit Phosgen in einem inerten organischen Lösungsmittel zu den entsprechenden Di- und Polyisocyanaten der Diphenylmethanreihe, dem MDI, umgesetzt werden. Das Molverhältnis von Roh-MDA zu Phosgen wird zweckmäßigerweise so bemessen, dass pro Mol N-H₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird zweckmäßigerweise so bemessen, dass die Reaktionsmischung einen Isocyanatgehalt von 2 bis 40 Gew.-%, vorzugsweise zwischen 5 uns 20 Gew.-%, bezogen auf das Gesamtgewicht der Reaktionsmischung, aufweist. Nach beendeter Phosgenierung wird aus der Reaktionsmischung das überschüssige Phosgen, das inerte organische Lösungsmittel oder Mischungen davon durch Destillation abgetrennt.

Aus dem gewonnenen Roh-MDI können die nach dem Stand der Technik bekannten Produkte der polymeren MDI-Reihe, enthaltend zwei- und höherkernige Di- und Polyisocyanate der Diphenylmethanreihe, sowie der monomeren MDI-Reihe, enthaltend zweikernige Diisocyanate der Diphenylmethanreihe, hergestellt werden, insbesondere hochviskose polymere MDI-Typen mit 80 bis 3.000 mPas bei 25°C, technisch reines 4,4'-MDI und/oder technisch reines 2,4'-MDI sowie deren Mischformen. Die Abtrennung dieser Produkte aus dem Roh-MDI kann gemäß dem Stand der Technik, beispielsweise durch Destillation, erfolgen. Diese Produkte eignen sich für den Einsatz als Rohstoffe für die Polyurethan-Herstellung in Form von Polymeren und Prepolymeren durch Reaktion mit Polyolen.

### Beispiele:

Allgemeine Versuchsvorschrift:

Zu 931 g Anilin werden bei 80°C unter Rühren innerhalb von 20 min 389 g einer 32,1 Gew.-%igen wässrigen Formaldehyd-Lösung getropft. Nach der Zugabe wird noch 5 min gerührt und bei 70 bis 80°C eine Phasentrennung vorgenommen. Die organische Phase, das Aminal, wird anschließend bei 45°C innerhalb von 20 min unter Rühren mit 114 g einer 32,0 gew.-%igen wässrigen Salzsäure versetzt. Nach 30 min Rühren bei 45°C wird das Reaktionsgemisch auf 60°C aufgeheizt und weitere 30 min bei 60°C gerührt. Anschließend wird die Reaktionstemperatur auf 104°C erhöht. Bei dieser Temperatur wird das Reaktionsgemisch für weitere 10 Stunden gerührt, um die Umsetzung zu vervollständigen.

500 g des so hergestellten sauren Reaktionsgemisches werden in einem Rührgefäß bei einer Temperatur von 90°C unter Rühren mit 62,3 g einer 33 gew.%igen Natronlauge versetzt. Das entstandene zweiphasige Gemisch wird ca. 5 min bei 90°C nachgerührt. Zur Phasentrennung bei 90°C wird der Rührer ausgeschaltet. Die Gehalte der einzelnen bzw. der Summe sämtlicher zwei- und höherwertigen Metallionen in der eingesetzten Salzsäure sowie die Ergebnisse der erfindungsgemäßen Beispiele und der Vergleichsbeispiele können der nachfolgenden Tabelle entnommen werden.

| | Gehalt an zwei- und höherwertigen Metallionen in der Salzsäure | | | | | Mulm |
|---|---|---|---|---|---|---|
| | Al | Ca | Fe | Mg | Summe | |
| | [Gew.-%] | [Gew.-%] | [Gew.-%] | [Gew.-%] | [Gew.-%] | |
| Vergleichsbeispiel 1 | 0,0010 | 0,0011 | 0,0020 | 0,0004 | > 0,001 | Ja |
| Vergleichsbeispiel 2 | 0,0005 | 0,00055 | 0,0010 | 0,0002 | > 0,001 | Ja |
| Vergleichsbeispiel 3 | 0,0002 | 0,0003 | 0,0005 | 0,0001 | > 0,001 | Ja |
| Beispiel 1 | 0,00013 | 0,00014 | 0,00025 | 0,00005 | < 0,001 | Nein |
| Beispiel 2 | < 0,00002 | < 0,00002 | < 0,00002 | < 0,00002 | < 0,001 | Nein |

Die Vergleichsbeispiele 1 bis 3 veranschaulichen die unerwünschte Bildung des Mulms bzw. die Ausbildung einer Mulmschicht an der Grenze zwischen der organischen und der wässrigen Phase. In den erfindungsgemäßen Beispielen 1 und 2 wird kein Mulm bzw. keine Mulmschicht gebildet.

## Patentansprüche

1. Verfahren zur Herstellung von Di- und Polyaminen der Diphenylmethanreihe, bei dem man Anilin und Formaldehyd in Gegenwart von Salzsäure umsetzt, **dadurch gekennzeichnet, dass** die eingesetzte Salzsäure weniger als 0,001 Gew.-% an zwei- und/oder höherwertigen Metallionen enthält.

2. Verfahren nach Anspruch 1, **dadurch gekennzeichnet, dass** die eingesetzte Salzsäure weniger als 0,0005 Gew.-% an zwei- und/oder höherwertigen Metallionen enthält.

3. Verfahren nach Anspruch 1, **dadurch gekennzeichnet, dass** die eingesetzte Salzsäure weniger als 0,0003 Gew.-% an zwei- und/oder höherwertigen Metallionen enthält.

4. Verfahren nach einem der Ansprüche 1 bis 3, **dadurch gekennzeichnet, dass** die eingesetzte Salzsäure die Metallionen Ca²⁺, Mg²⁺, Al³⁺ und/oder Fe²⁺ jeweils in Konzentrationen von kleiner 0,0003 Gew.-% enthält.

5. Verfahren nach einem der Ansprüche 1 bis 4, **dadurch gekennzeichnet, dass** die Umsetzung von Anilin mit Formaldehyd in Gegenwart von Salzsäure bei einem Protonierungsgrad von kleiner 15% durchgeführt wird.

6. Verfahren zur Herstellung von Di- und Polyisocyanaten der Diphenylmethanreihe, bei dem man Di- und Polyamine der Diphenylmethanreihe nach einem der Ansprüche 1 bis 5 herstellt, und anschließend die Di- und Polyamine der Diphenylmethanreihe mit Phosgen zu Di- und Polyisocyanaten der Diphenylmethanreihe umsetzt.

## Claims

1. Process for the preparation of di- and polyamines of the diphenylmethane series in which aniline and formaldehyde are reacted in the presence of hydrochloric acid, **characterized in that** the hydrochloric acid employed contains less than 0.001 wt.% of metal ions which are divalent and/or more than divalent.

2. Process according to claim 1, **characterized in that** the hydrochloric acid employed contains less than 0.0005 wt.% of metal ions which are divalent and/or more than divalent.

3. Process according to claim 1, **characterized in that** the hydrochloric acid employed contains less than 0.0003 wt.% of metal ions which are divalent and/or more than divalent.

4. Process according to one of claims 1 to 3, **characterized in that** the hydrochloric acid employed contains the metal ions Ca²⁺, Mg²⁺, Al³⁺ and/or Fe²⁺ in each case in concentrations of less than 0.0003 wt.%.

5. Process according to one of claims 1 to 4, **characterized in that** the reaction of aniline with formaldehyde in the presence of hydrochloric acid is carried out with a degree of protonation of less than 15 %.

6. Process for the preparation of di- and polyisocyanates of the diphenylmethane series, in which di- and polyamines of the diphenylmethane series are prepared according to one of claims 1 to 5, and the di- and polyamines of the diphenylmethane series are then reacted with phosgene to give di- and polyisocyanates of the diphenylmethane series.

## Revendications

1. Procédé de préparation de di- et polyamines de la série du diphénylméthane, dans lequel on fait réagir l'aniline et le formaldéhyde en présence d'acide chlorhydrique, **caractérisé en ce que** l'acide chlorhydrique mis en oeuvre contient moins de 0,001% en poids d'ions métalliques bivalents et/ou de valence supérieure.

2. Procédé selon la revendication 1, **caractérisé en ce que** l'acide chlorhydrique mis en oeuvre contient moins de 0,0005% en poids d'ions métalliques bivalents et/ou de valence supérieure.

3. Procédé selon la revendication 1, **caractérisé en ce que** l'acide chlorhydrique mis en oeuvre contient moins de 0,0003% en poids d'ions métalliques bivalents et/ou de valence supérieure.

4. Procédé selon l'une des revendications 1 à 3, **caractérisé en ce que** l'acide chlorhydrique mis en oeuvre contient les ions métalliques Ca²⁺, Mg²⁺, Al³⁺ et/ou Fe²⁺, chaque fois en des concentrations inférieures à 0,0003% en poids.

5. Procédé selon l'une des revendications 1 à 4, **caractérisé en ce que** la réaction de l'aniline avec le formaldéhyde en présence d'acide chlorhydrique est réalisée à un taux de protonation inférieur à 15%.

6. Procédé de préparation de di- et polyisocyanates de la série du diphénylméthane, dans lequel on prépare des di- et polyamines de la série du diphénylméthane selon l'une des revendications 1 à 5, et ensuite, on fait réagir les di- et polyamines de la série du diphénylméthane avec le phosgène pour obtenir les di- et polyisocyanates de la série du diphénylméthane.
